**Europäisches Patentamt**

**European Patent Office**

**Office européen des brevets**

(19)

Veröffentlichungsnummer: **0 124 844**
A2

(12)

# EUROPÄISCHE PATENTANMELDUNG

(21) Anmeldenummer: 84104836.6

(22) Anmeldetag: 30.04.84

(51) Int. Cl.³: **G 01 N 29/00**

---

(30) Priorität: 04.05.83 DE 3316171

(43) Veröffentlichungstag der Anmeldung:
14.11.84 Patentblatt 84/46

(84) Benannte Vertragsstaaten:
CH FR GB IT LI

(71) Anmelder: **Mahlo GmbH & Co. KG**
**Donaustrasse 12**
**D-8424 Saal/Donau(DE)**

(72) Erfinder:
**Der Erfinder hat auf seine Nennung verzichtet**

(74) Vertreter: **Reinländer & Bernhardt Patentanwälte**
**Orthstrasse 12**
**D-8000 München 60(DE)**

---

(54) Verfahren und Vorrichtung zur Kontrolle der Farbaufnahmefähigkeit von Textilien.

Zur Kontrolle der Farbaufnahmefähigkeit einer textilen Ware mittels Luft oder Schall wird eine Bahn kontinuierlich zwischen Sender und Empfänger durchgeführt und werden an verschiedenen Stellen der Bahn ermittelte Meßwerte miteinander verglichen.

Fig. 2

EP 0 124 844 A2

64/31

Mahlo GmbH + Co. KG
Donaustr. 12
8424  Saal/Donau


Verfahren und Vorrichtung zur Kontrolle
der Farbaufnahmefähigkeit von Textilien

Ein großes und für den Qualitätsausfall entscheidendes
Problem beim Färben von Textilien ist der ungleichmäßige
Farbausfall über Breite und Länge der zu färbenden Stücke.
Farbunterschiede, sogenannte Farbabläufe, zwischen der
Mitte der Bahn und den Kantenzonen sind nicht selten; desgleichen treten solche Unterschiede auch häufig zwischen
Anfang und Ende einer Textilbahn auf. Bei der Konfektionierung derartiger gefärbter Artikel äußern sich dann solche
Farbabweichungen in nicht akzeptierbaren Farbunterschieden,
z.B. innerhalb eines Kleidungsstückes.

Die Färbefehler dieser Art können verschiedene Ursachen
haben. Je nach angewandten Färbeverfahren sind maschinentechnische Fehler, Bedienungsfehler, insbesondere aber
auch Ungleichmäßigkeiten in der zu färbenden Rohware möglich.
Ein Grund für Farbabläufe ist die Aufbringung unterschiedlich großer Farbflottenmengen auf die Ware, bedingt etwa
durch nicht gleichmäßige Abquetschung der Ware selbst.
Es sind deshalb Einrichtungen bekannt, die die Kontrolle
der über die Breite der Ware verteilten Wassermenge zu
kontrollieren erlauben, z.B. mit Hilfe von Feuchtigkeitsmeßgeräten nach dem Mikrowellen-Absorptionsverfahren (DE-PS
26 55 973). Zur Überwachung der Farbgleichmäßigkeit wurden
auch farbmetrische Meßgeräte entwickelt, die bereits an
der laufenden Warenbahn, aber natürlich nach der Trocknung
der Farbe, die Farben objektiv zu messen gestatten.

Die Messung der Verteilung der wässrigen Farbflotte und
die mögliche Regelung dieser Verteilung berücksichtigt
jedoch nur eine der vielfältigen Ursachen für die Farbungleichmäßigkeit und - wie die Erfahrungen der Färber
lehren - nicht einmal die entscheidende. Dagegen bringt
die Rohware selbst sehr häufig Eigenschaften mit, die eine
unterschiedliche Farbflottenaufnahme verursachen. Solche
sind beispielsweise ein ungleichmäßiges Flächengewicht,
vor allem aber auch nicht gleichmäßige Farbaufnahmeeigenschaften, hervorgerufen beispielsweise durch Schwankungen
in der Struktur der Textilien, Faserrauhigkeit, der Größe
und der Verteilung der Kapillaren zwischen den Fasern,
des dadurch bedingten hygroskopischen Verhaltens und dgl..

Es ist deshalb für den Färber sehr wichtig, die Farbaufnahmeeigenschaften eines angelieferten ungefärbten Stückes
bereits vor der Färbung zu kennen, damit besonders ungleichmäßige Partien von vornherein z.B. für eine Uni-Färbung
gar nicht erst vorgesehen werden. Solche Stücke können
dagegen zum Druck oder zu anderer Verwendung disponiert
werden, bei der Farbabläufe keine gravierende Rolle spielen.

Der Erfindung liegt deshalb die Aufgabe zugrunde, ein Verfahren und eine Vorrichtung zur Kontrolle von Textilien
zu schaffen, mit denen durch Farbunterschiede bedingte
Ausfälle der textilen Ware ohne Schädigung derselben vermieden werden. Gelöst wird diese Aufgabe durch die Merkmale
der Ansprüche.

Der Erfindung liegt die Erkenntnis zugrunde, daß eine
Relation zwischen der Farbaufnahme von Textilien und ihrer
Durchlässigkeit besteht. Unterschiedliche Durchlässigkeiten
einer Warenbahn gegenüber Luft oder Schall     rufen
ebenfalls ungleichmäßige Farbverteilungen hervor, wobei
solche Durchlässigkeitsunterschiede nicht unbedingt auf
Flächengewichtsunterschieden beruhen. Vielmehr sind für
derartige Durchlässigkeitsverschiedenheiten Unterschiede

0124844

in der Struktur des Textils verantwortlich, die sich in Zahl, Art, Form und Größe der zwischen den Fasern entstehenden Zwischenräume (Kapillare) und der Zwischenräume zwischen den Fäden, im Aussehen der Oberfläche der Garne und ähnlichen textilmechanischen Merkmalen äußern. Die Ursachen für solche Unterschiede sind sowohl bei der Stoffherstellung (Weberei, Strickerei) als auch bereits bei den verwendeten Garnen selbst zu suchen.

Zum Zwecke der erfindungsgemäßen Kontrolle von Textilien auf ihre Anfärbbarkeit hin wird Luft oder Schallenergie an einer oder mehreren Stelle(n) der Bahn (Kanten, Mitte) oder mit Hilfe eines oder mehrerer quer über die Bahn traversierender Meßköpfe oder durch bandförmige, sich über die ganze Breite erstreckende Meßaggregate auf das Textil appliziert. Mit geeigneten Empfängern wird die Menge der durch das Textil durchtretenden Energien gemessen. Dies kann mit Empfängern für Luftdurchfluß, Luftdruck oder Schallamplituden geschehen. Die an den verschiedenen Stellen der Bahn gemessenen Signale werden in einer Vergleichseinrichtung miteinander verglichen; ein Rechner kann die prozentualen Abweichungen errechnen; ein Drucker oder ein Schreiber kann die Gleichmäßigkeit anzeigen.

Die Messung kann aber auch mittels Reflexion erfolgen, indem die von der Warenbahn reflektierte Luft, d.h. mit dem an der einen Seite der Bahn angeordneten Meßorgan der Druck der auf die Warenbahn gerichteten Luft bzw. der durch die Warenbahn erzeugte Rückdruck kontrolliert wird. Ein Meßorgan an der anderen Seite der Warenbahn entfällt dann. Auch beim Kontrollieren der durch die Warenbahn angesaugten Luft ist nur an einer Seite der Warenbahn ein Meßorgan erforderlich.

An sich ist es bekannt, die Durchlässigkeit bzw. den Strömungswiderstand einer textilen Ware gegenüber Luft zu messen, so die Wasserdurchlässigkeit von Regenbekleidung und den Luftwiderstand von Fallschirmseide. Diese Messungen erfolgen
aber stationär, so daß dadurch nicht über eine große Fläche
Unterschiede der Durchlässigkeit ermittelt werden können.

Im folgenden wird anhand eines Beispiels beschrieben, wie
eine derartige Meßanordnung ausgeführt werden kann.

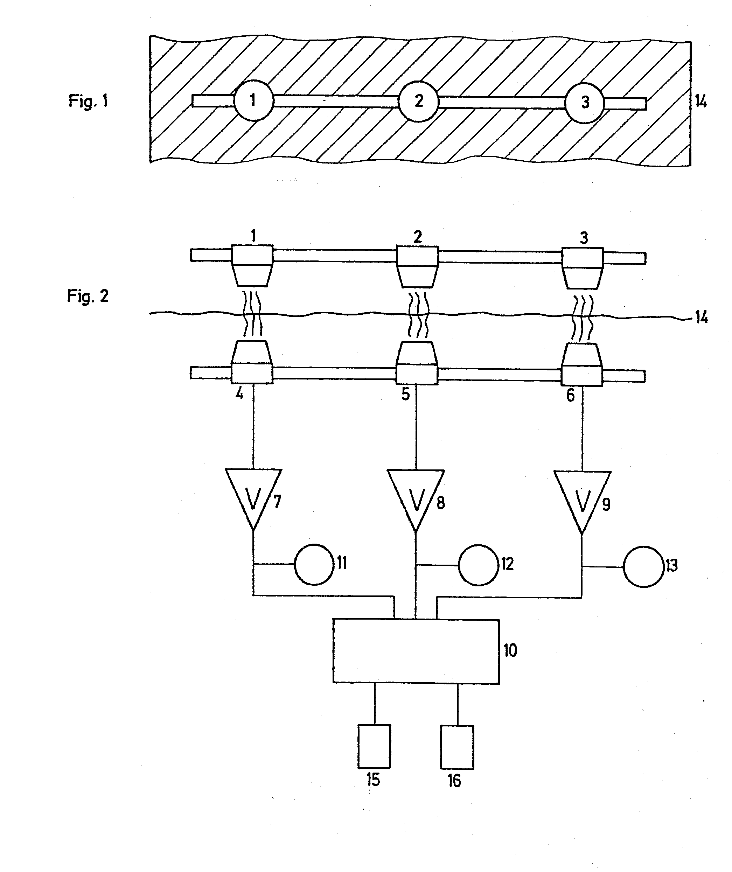

In Fig. 1 ist in Draufsicht und in Fig. 2 im Schnitt ein
Schema der Meßanordnung zur Messung von Farbaufnahmeeigenschaften der Waren dargestellt.

In den Sendern 1 bis 3 wird Schallenergie (Ultraschall)
erzeugt und auf die textile Warenbahn 14 aufgestrahlt.
Die Empfänger 4 bis 6 können (Schall)druckmesser sein.
Sie wandeln die durch das Textil hindurchtretenden
Energien in elektrische Signale um. Die empfangenen
Energien werden mit den jeweils zugehörigen Sendeenergien
verglichen. Daraus wird die Dämpfung bestimmt, die
ein Maß für die Durchlässigkeit des Prüfguts ist. Außerdem werden in einer                        Schaltung 10
die Dämpfungen der drei Strecken (1 - 4, 2 - 5 und
3 - 6) miteinander verglichen, um Ungleichmäßigkeiten
in der Durchlässigkeit festzustellen. Mit den Anzeigegeräten 11, 12 und 13 wird schließlich die Durchlässigkeit des Prüfguts an drei Stellen (Mitte und beide
Kanten) angezeigt. Sie kann gleichzeitig ausgedruckt (15)
oder geschrieben(16) werden; die Drucker- oder Schreiber-
Protokolle werden zweckmäßigerweise mit den textilen
Stücken als Unterlagen für die Färberei weitergegeben.
7, 8 und 9 sind Verstärker.
Da zur Erzielung des erfindungsgemäßen Effektes nur
relative Meßwerte, also der Vergleich gegenüber einem
vorgegebenen Standard über die Bahnlänge interessieren,
werden auch nur genügend gleichartige Meßanordnungen
benötigt, nicht aber reproduzierbare absolute Meßwerte.
Die Auswahl des anzuwendenden Meßmediums richtet sich
nach den Gegebenheiten und Eigenschaften des textilen Prüfguts, die allerdings erheblich unterschiedlich sein
können. So werden dichte und weitgehend undurchscheinende
Textilien mit Luft oder Schall als Medium geprüft.

Wegen der geforderten Gleichheit der Eigenschaften der
einzelnen Meßstrecken empfiehlt sich u.U. die Verwendung
nur eines Meßaggregats, das aber quer zur Bahn bewegt wird
und dabei ein lückenloses Profil der Durchlässigkeitsverhältnisse liefert. Auch zwei oder mehr symmetrisch zur
Mitte der Bahn bewegte Meßaggregate sind denkbar, deren
Signale ständig paarweise miteinander verglichen werden.

0124844

64/31

Mahlo GmbH + Co. KG
Donaustraße 12
8424  Saal/Donau

P a t e n t a n s p r ü c h e

1. Verfahren zur Kontrolle der Farbaufnahmefähigkeit einer textilen Ware, insbesondere dichten Ware, mittels mechanischer Energie, insbesondere Luft, mit mindestens einem Meßorgan an mindestens einer Seite der Ware, dadurch gekennzeichnet, daß eine Bahn der Ware kontinuierlich an dem Meßorgan vorbeigeführt wird und daß die an verschiedenen Stellen in Längs- und/oder Querrichtung der Bahn ermittelten Meßwerte des Drucks und/oder der Durchflußmenge miteinander verglichen werden.

2. Verfahren nach Anspruch 1, dadurch gekennzeichnet, daß die Luft auf die Bahn gedrückt wird.

3. Verfahren nach Anspruch 1, dadurch gekennzeichnet, daß die Luft durch die Bahn gesaugt wird.

4. Verfahren nach Anspruch 1, dadurch gekennzeichnet, daß Schallenergie durch die Bahn gesandt wird.

5. Verfahren nach Anspruch 4, dadurch gekennzeichnet, daß Ultraschallenergie durch die Bahn gesandt wird.

6. Vorrichtung zur Durchführung des Verfahrens nach Anspruch 1, dadurch gekennzeichnet, daß der Sender (1, 2, 3) und der Empfänger (4, 5, 6) quer zur Bahn (14) bewegbar sind.

7. Vorrichtung nach Anspruch 6, dadurch gekennzeichnet, daß zwei oder mehrere geradzahlige Sender/Empfänger symmetrisch zur Mitte der Bahn hin- und herbewegbar sind.

1/1

Fig. 1

Fig. 2